# EUROPÄISCHE PATENTSCHRIFT

(11) **EP 2 477 583 B1**
(45) Veröffentlichungstag und Bekanntmachung des Hinweises auf die Patenterteilung: **08.04.2015**
(21) Anmeldenummer: 09744929.2
(22) Anmeldetag: 16.09.2009
(51) Int. Cl.: A61F 2/915, A61F 2/91

(54) **STENT MIT DEHNELEMENTEN**
STENT HAVING EXPANDABLE ELEMENTS
STENT POURVU D'ÉLÉMENTS EXTENSEURS

(43) Veröffentlichungstag der Anmeldung: 25.07.2012
(73) Patentinhaber: Bentley InnoMed GmbH, 72379 Hechingen (DE)
(72) Erfinder: OBRADOVIC , Milisav, 79539 Lörrach (DE)
(74) Vertreter: Bähring, Albrecht
(86) Internationale Anmeldenummer: PCT/DE2009/001306
(87) Internationale Veröffentlichungsnummer: WO 2011/032526

(56) Entgegenhaltungen:
- EP-A1- 1 958 598
- WO-A2-2008/008291
- DE-A1- 10 103 000
- US-A1- 2002 010 507
- US-A1- 2003 014 102
- US-B1- 6 776 794

## Beschreibung

Die Erfindung betrifft ein medizinisches Implantat zur Aufweitung und Stützung eines Körpergefäßes von dessen Innenseite her (="Stent"), das in einem kontrahierten Zustand minimal-invasiv in das Körpergefäß einsetzbar, dort positionierbar und in einen expandierten Zustand überführbar ist, wobei das Implantat röhrenförmig aus länglichen, eine Wand des Implantats bildenden Stegen aufgebaut ist, und wobei die Stege zumindest abschnittsweise derart plastisch verformbar sind, dass das Implantat im expandierten Zustand in radialer Richtung quer zur Längsachse der Stege dauerhaft aufgeweitet bleibt. WO-A-2008/008291 beschreibt die technischen Merkmale des Oberbegriffs aus Anspruch 1.

Derartige Stents sind seit langem bekannt, beispielsweise auch aus der DE 101 05 160 B4.

Ein solcher Stent weist mindestens eine geometrische Struktur auf, die es erlaubt, dass er - meist über mehrere Stufen - aus seinem kontrahierten Zustand in einen aufgeweiteten Zustand überführt werden kann (="Redilatierung"). Allerdings kann der lichte Querschnitt des Stents ausgehend vom ursprünglichen Minimalquerschnitt jeweils nur auf ein vorbestimmtes Maximalmaß aufgedehnt werden. Eine weitere radiale Dilatierung ist nicht möglich, weil sich sonst der Stent undefiniert verformen und anschließend brechen würde. Für jede Gefäßgröße wird daher eine bestimmte Stentgröße hergestellt und verwendet, damit eine optimale radiale Haltekraft des Stents im Einzelfall sichergestellt werden kann.

Bisher mögliche und übliche Aufweitungsgruppen sehen Querschnittsvergrößerungen des Stents (gemessen jeweils in Millimetern des lichten Durchmessers) auf 4 oder 6, auf 6 oder 8, auf 8 oder 10, auf 10 oder 12 vor. Noch größere Aufweitungen sind unüblich und werden in der Regel nur für Spezialanwendungen eingesetzt, wobei dann allerdings von bereits viel größeren Ausgangs- bzw. Minimalquerschnitten ausgegangen wird, also etwa Aufweitungen von 16mm auf 20mm Durchmesser.

Eines der Hauptprobleme bei der Verwendung derartiger Stents liegt in den Anwendungen bei kleinen Kindern, insbesondere im Bereich der Kinder- und Säuglingskardiologie: Hier muss man aufgrund der geringen Gefäßgrößen der kleinen Patienten zunächst immer mit Stents sehr geringer Ausgangsquerschnitte arbeiten. Danach wächst das Kind jedoch zum Jugendlichen und schließlich zum Erwachsenen heran, die Gefäße vergrößern sich gegenüber dem Zeitpunkt der ersten Implantation enorm und die maximale Ausweitbarkeit des jeweils eingesetzten Stents ist sehr schnell erreicht, so dass der Stent im Verlauf in der Regel mindestens einmal gegen ein größeres Modell ausgetauscht werden muss. Dieser Austausch ist natürlich jedes Mal mit einer Operation verbunden, was für den heranwachsenden Patienten sehr belastend ist, selbst wenn minimal-invasive Techniken angewandt werden können.

Aufgabe der vorliegenden Erfindung ist es demgegenüber, eine gattungsgemäße Anordnung der eingangs beschriebenen Art mit möglichst einfachen technischen Mitteln unaufwändig und kostengünstig dahin gehend zu verbessern, dass über das bislang baubedingte Maximalmaß der Redilatierung hinaus ein ganz erheblich größerer Bereich von Aufweitungen des Querschnitts des röhrenförmigen Implantats in Radialrichtung ausgehend vom minimal kontrahierten Ursprungszustand bis zum maximal redilatierten Endzustand ermöglicht wird, der es dann letztlich erlaubt, einen im Kindesalter eingesetzten Stent - wenn medizinisch möglich - bis zum Erwachsenenalter im Körper des Patienten zu belassen ("mitwachsender Kinderstent").

Erfindungsgemäß wird diese Aufgabe auf ebenso überraschend einfache wie wirkungsvolle Weise dadurch gelöst, dass zumindest einer der Stege ein Dehnelement aufweist, welches einen Stegabschnitt innerhalb dieses Steges bildet, in dem das Dehnelement im kontrahierten Zustand teilweise geometrisch quer zur Längsachse des Steges verläuft, wobei der Verlauf des Dehnelements bezüglich dieser Längsachse mehrere Richtungsänderungen aufweist, und dass Material, Dicke und Stegbreite des Dehnelements derart gewählt sind, dass durch Krafteinleitung in Radialrichtung des röhrenförmigen Implantats das Dehnelement durch plastische Streckung in einen dauerhaft expandierten Zustand versetzt werden kann, in welchem der das Dehnelement bildende Stegabschnitt eine größere geometrische Ausdehnung quer zur Längsachse und eine geringere Ausdehnung in Richtung der Längsachse aufweist als im kontrahierten Zustand. Auf diese Weise lassen sich Redilatierungen in einem sehr weiten Bereich erzielen, die ganz erheblich über das bisher baubedingte Maximalmaß herkömmlicher Stents hinausgehen.

Ganz besonders bevorzugt sind Ausführungsformen des erfindungsgemäßen Implantats, bei denen das Dehnelement aufgrund seiner Materialeigenschaften und seiner Geometrie so ausgebildet ist, dass eine Ausdehnung des Querschnittdurchmessers des röhrenförmigen Implantats in Radialrichtung vom minimal kontrahierten bis zum maximal redilatierten Zustand von 2mm auf 18mm, vorzugsweise von 4mm auf 16mm, oder von 6mm auf 24mm, vorzugsweise von 6mm auf 20mm, ermöglicht wird. Damit können in der Praxis die normalerweise vorkommenden Bereiche des menschlichen Wachstums vom Kleinkind bis zum Erwachsenen abgedeckt werden.

Eine fertigungstechnisch besonders einfach und kompakt herstellbare Klasse von Ausführungsformen des erfindungsgemäßen Implantats zeichnet sich dadurch aus, dass das Dehnelement im kontrahierten Zustand zumindest abschnittsweise einen zickzackförmigen, schlangenlinienförmigen und/oder mäanderförmigen geometrischen Verlauf aufweist. Je nach Anzahl der Zickzack-Strukturen - die vorteilhaft in einander entgegengesetzten Richtungen angeordnet sein können - kann die Aufweitbarkeit des Stents, insbesondere im Vergleich zu herkömmlichen Stents nach dem Stand der Technik, ziemlich groß werden. Zudem weist ein erfindungsgemäßer Stent mit derartig geformten Dehnelementen aufgrund dieser Struktur unabhängig von der Redilationsstufe eine besonders große Radialkraft auf.

In der Praxis bewährt sich aber auch eine andere Klasse von Ausführungsformen der Erfindung, bei denen das Dehnelement im kontrahierten Zustand zumindest abschnittsweise einen spiralförmigen geometrischen Verlauf aufweist.

Besonders vorteilhaft sind Weiterbildungen, die sich dadurch auszeichnen, dass das Dehnelement im kontrahierten Zustand zumindest abschnittsweise den geometrischen Verlauf einer Doppelspirale aufweist, die sich ausgehend von einem gemeinsamen Mittelpunkt in zwei entgegengesetzten Drehrichtungen krümmt. Weiterbildungen dieser Ausführungsformen sind möglich, bei denen die Spirale eine runde, insbesondere kreisrunde, oder eine dreieckige oder eine viereckige, insbesondere quadratische, Außenkontur aufweist.

Das Dehnelement des erfindungsgemäßen Implantats weist im kontrahierten Zustand eine Engstelle auf, deren Stegbreite geringer ist als die Stegbreiten der anschließenden Stegabschnitte.

Die Stegbreite der Engstelle ist höchstens zwei Drittel, vorzugsweise höchstens halb so groß wie die Stegbreiten der anschließenden Stegabschnitte.

Die Engstelle ist als Sollbruchstelle für das Dehnelement ausgelegt die bei zu großer Krafteinleitung in Radialrichtung des röhrenförmigen Implantats bricht, eine weitere Möglichkeit zur Redilatation eröffnet und dadurch eine weitere Expansion des Dehnelements verhindert. In diesem Fall wird dann zwar der entsprechende Steg des Implantats in Längsrichtung unterbrochen. Trotz dieser Unterbrechung kann aber in den meisten praktischen Anwendungsfällen auf die Implantation eines neuen Stents verzichtet werden, da sich das behandelte Gefäß nach einer derartigen Wachstumsperiode in der Regel bereits genügend stabilisiert hat, so der verbleibende gebrochene Stent als Stütze ausreicht.

Wie bereits per se aus dem Stand der Technik bekannt ist, können zwischen Paaren von in Richtung der Längsachse des Implantats benachbarten Stegen Verbinderelemente angeordnet sein, die bei Krafteinleitung in Längsrichtung dieser Stege eine dauerhafte plastische Vergrößerung des Implantats in axialer Richtung bewirken.

Bevorzugt ist eine Ausführungsform des erfindungsgemäßen Implantats, bei der mehrere Dehnelemente pro Stent vorgesehen sind, wodurch sich die Flexibilität und die Anwendungsbandbreite des Implantats erhöhen.

Bei einer Klasse von Weiterbildungen dieser Ausführungsform weisen die Dehnelemente untereinander die gleichen mechanischen Eigenschaften auf und sind insbesondere geometrisch gleich aufgebaut, wodurch sich ein einheitlicher Herstellungsprozess anwenden lässt.

Alternativ können bei einer anderen Klasse von Weiterbildungen die Dehnelemente untereinander unterschiedliche mechanischen Eigenschaften aufweisen. Dies ermöglicht eine große Bandbreite von individuell auf spezielle Probleme des Patienten angepassten Designs der erfindungsgemäße Implantate.

Die unterschiedlichen mechanischen Eigenschaften der Dehnelemente können bei Varianten dieser Weiterbildungen dadurch erreicht werden, dass die Dehnelemente zwar geometrisch gleich aufgebaut sind, was ein einheitliches Herstellungsverfahren begünstigt, jedoch unterschiedliche Stegbreiten aufweisen.

Eine andere Variante der Erfindung hingegen zeichnet sich dadurch aus, dass die Dehnelemente geometrisch unterschiedlich aufgebaut sind. Dies lässt wiederum eine große Vielfalt an unterschiedlichen, auf individuelle Problemlösungen abzielende Designs der Implantate zu.

Bei bevorzugten Weiterbildungen der oben beschriebenen Ausführungsform können mehrere Dehnelemente in Längsrichtung des Implantats hintereinander angeordnet sein.

Vorteilhaft sind auch Varianten, bei denen mehrere Dehnelemente azimutal, insbesondere symmetrisch, über den Umfang des Implantats verteilt angeordnet sind.

Ganz besonders bevorzugt sind Ausführungsformen des erfindungsgemäßen Implantats, bei welchen die Stege einschließlich der Dehnelemente aus einem rohrförmigen Ausgangsteil, vorzugsweise durch Laserschneiden, hergestellt sind. Hiermit lassen sich auch besonders feine Strukturen und Konturen in reproduzierbarer Qualität und mittlerweile auch in großen Mengen erzeugen.

Das erfindungsgemäße Implantat kann zumindest im Bereich des Dehnelements aus einem Material unter Verwendung von Chrom und/oder Kobalt und/oder Platin und/oder einer Legierung der genannten Metalle und/oder einer Edelstahllegierung hergestellt sein.

Vorteilhaft für viele Anwendungen ist es auch wenn das Implantat zumindest im Bereich des Dehnelements aus einem Material mit Formgedächtnis (=memory effect), insbesondere aus Nitinol hergestellt ist, welches etwa durch Einwirkung von Wärme eine definierte - und bei Aufrechterhaltung der thermischen Bedingungen auch dauerhafte - Strukturänderung erfährt.

Zumindest Teile des Implantats können auch aus biokompatiblen Kunststoffen, insbesondere Silikon oder Polytetrafluorethylen (PTFE), und/oder aus Faserverbund-Werkstoffen, insbesondere Kohlefasern hergestellt sein.

Des Weiteren kommen als Werkstoffe für das erfindungsgemäße Implantat oder zumindest von Teilen desselben Titan und/oder Gold und/oder Tantal und/oder eine Legierung der genannten Metalle in Frage.

Für spezielle Anwendungsfälle kann es aber auch von Nutzen sein, wenn zumindest Teile des Implantats aus einem Keramikmaterial hergestellt sind.

Vorteilhaft sind schließlich auch Ausführungsformen der Erfindung, die sich dadurch auszeichnen, dass zumindest abschnittsweise auf der Oberfläche des Implantats eine biologisch aktive Beschichtung, insbesondere eine wachstumshemmende und/oder eine wachstumsfördernde und/oder eine antibakteriell wirkende Beschichtung, vorgesehen ist.

Weitere Merkmale und Vorteile der Erfindung ergeben sich aus der nachfolgenden detaillierten Beschreibung von Ausführungsbeispielen der Erfindung anhand der Figuren der Zeichnung, die erfindungswesentliche Einzelheiten zeigt, sowie aus den Ansprüchen. Die einzelnen Merkmale können je einzeln für sich oder zu mehreren in beliebigen Kombinationen bei Varianten der Erfindung verwirklicht sein.

In der schematischen Zeichnung sind Ausführungsbeispiele der Erfindung dargestellt, welche in der nachfolgenden Beschreibung näher erläutert werden.

Im Einzelnen zeigen:
- Fign. 1a-d: eine Ausführungsform des erfindungsgemäßen Implantats mit zwei in Längsrichtung hintereinander angeordneten, schlangenförmigen Dehnelementen in schematischer räumlicher Darstellung, nämlich 1a) von der Seite, 1b) von oben in Längsrichtung der Stege, 1c) räumlich schräg und 1d) vergrößert von der Seite;
- Fig. 2: eine Ausführungsform mit nur einem Dehnelement sowie im Detail verschiedene Phasen der Aufdehnung des Implantats; und
- Fign. 3a-c: Ausführungsformen von erfindungsgemäßen Dehnelementen in Form von Doppelspiralen, nämlich 3a) dreieckig, 3b) rund und 3c) viereckig.

Die in den Figuren der Zeichnung schematisch dargestellten Ausführungsformen des erfindungsgemäßen medizinischen **Implantats 10; 20** zur Aufweitung und Stützung eines Körpergefäßes von dessen Innenseite her (="Stent"), sind jeweils röhrenförmig aus länglichen, eine Wand des Implantats 10; 20 bildenden **Stegen 11,11',11"; 21,21',21"** aufgebaut, die zumindest abschnittsweise derart plastisch verformbar sind, dass das Implantat 10; 20 nach Redilatierung in radialer Richtung quer zur Längsachse der Stege 11,11',11"; 21,21',21" dauerhaft aufgeweitet bleibt. Um eine gegenüber bekannten Stents erheblich größere Aufweitbarkeit des Implantats 10; 20 zu erreichen, enthält zumindest einer der Stege 11; 21 ein **Dehnelement 12,12'; 22; 32a,32b,32c,** welches einen Stegabschnitt innerhalb dieses Steges 11; 21 bildet, in dem das Dehnelement 12,12'; 22; 32a,32b,32c im kontrahierten Zustand teilweise geometrisch quer zur Längsachse des Steges 11; 21 verläuft, wobei der Verlauf des Dehnelements 12,12'; 22; 32a,32b,32c bezüglich dieser Längsachse einige Richtungsänderungen aufweist. Material, Dicke und Stegbreite des Dehnelements 12,12'; 22; 32a,32b,32c sind derart gewählt, dass durch Krafteinleitung in Radialrichtung des röhrenförmigen Implantats 10; 20 das Dehnelement 12,12'; 22; 32a,32b,32c durch plastische Streckung in einen dauerhaft expandierten Zustand versetzt werden kann, in welchem der das Dehnelement 12,12'; 22; 32a,32b,32c bildende Stegabschnitt eine größere geometrische Ausdehnung quer zur Längsachse und eine geringere Ausdehnung in Richtung der Längsachse aufweist als im kontrahierten Zustand.

Sämtliche in der Zeichnung dargestellten Ausführungsformen der Erfindung stimmen auch darin überein, dass die Dehnelemente 12,12'; 22; 32a,32b,32c jeweils eine als Sollbruchstelle ausgelegte **Engstelle 13,13'; 23; 33a,33b,33c** besitzen, die bei zu großer Krafteinleitung in Radialrichtung des röhrenförmigen Implantats 10; 20 bricht, eine weitere Möglichkeit zur Redilatation eröffnet und dadurch eine weitere Expansion des Dehnelements 12,12'; 22; 32a,32b,32c verhindert.

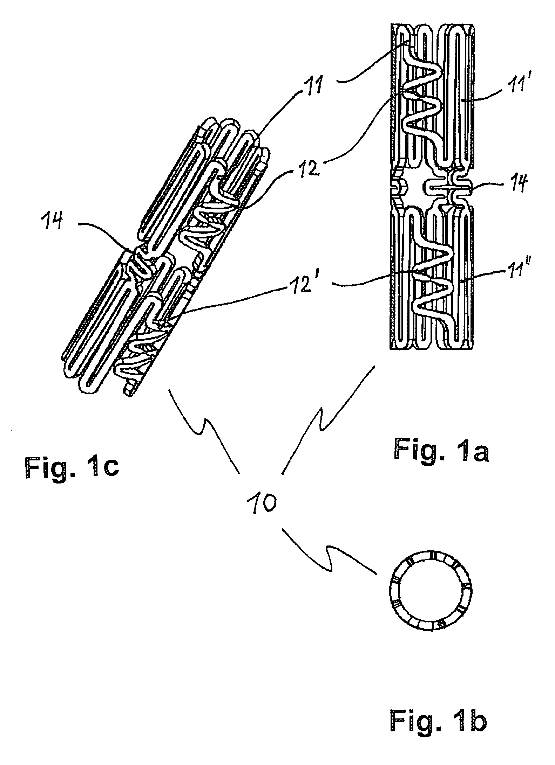

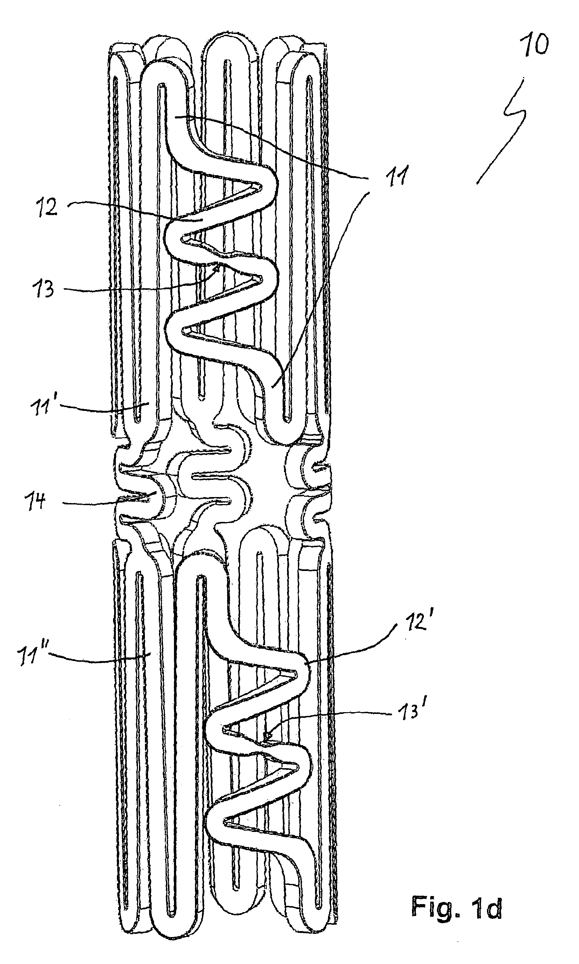

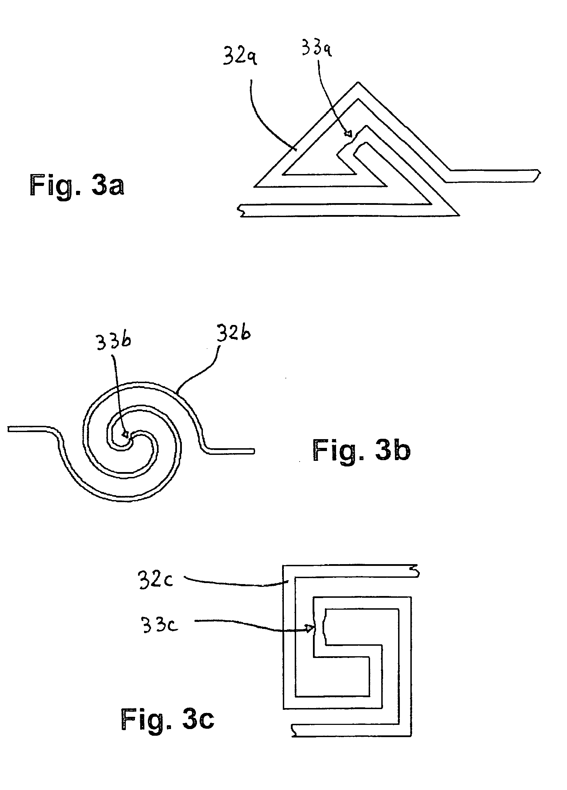

Die in den Figuren 1a bis 2 dargestellten Dehnelemente 12,12'; 22 weisen jeweils einen schlangenlinienförmigen geometrischen Verlauf auf, während die - sehr schematisch - in den Figuren 3a bis 3c dargestellten Ausführungsformen erfindungsgemäßer Dehnelemente 32a, 32b, 32c jeweils den geometrischen Verlauf einer Doppelspirale aufweisen, die sich ausgehend von einem gemeinsamen Mittelpunkt in zwei entgegengesetzten Drehrichtungen krümmt. Bei dem Ausführungsbeispiel in Fig. 3a ist diese Doppelspirale dreieckig, in Fig. 3b rund und in Fig. 3c viereckig. Denkbar sind auch andere geometrische Formgebungen, etwa anstelle der abgerundeten Schlangenlinien zickzackförmige oder mäanderförmige Verläufe der Dehnelemente, die jedoch in der Zeichnung nicht dargestellt sind.

Die in den Figuren 1a bis 1d dargestellte Ausführungsform zeichnet sich außerdem dadurch aus, dass mehrere Dehnelemente 12, 12' - hier genau zwei - in Längsrichtung des Implantats 10 hintereinander angeordnet sind. Diese sind im vorliegenden Ausführungsbeispiel geometrisch gleich aufgebaut und können die gleichen, aber auch unterschiedliche mechanischen Eigenschaften aufweisen.

Zwischen einigen Paaren von in Richtung der Längsachse des Implantats 10 benachbarten Stegen 11', 11" sind bei dieser Ausführungsform **Verbinderelemente 14** angeordnet, die bei Krafteinleitung in Längsrichtung dieser Stege 11', 11" eine dauerhafte plastische Vergrößerung des Implantats 10 in axialer Richtung bewirken. Diese Verbinderelemente 14 erfüllen also eine völlig andere Funktion als die erfindungsgemäßen Dehnelemente 12, 12' und bilden auch keinen Stegabschnitt innerhalb eines der Stege 11, 11', 11". Vielmehr sitzen sie axial zwischen benachbarten Stegen 11', 11" und können auch keine radiale Aufweitung des Implantats 10 bewirken, sondern nur eine axiale Verlängerung.

Bei in der Zeichnung nicht dargestellten Ausführungsformen des erfindungsgemäßen Implantats können auch mehrere Dehnelemente azimutal über den Umfang des Implantats verteilt angeordnet sein.

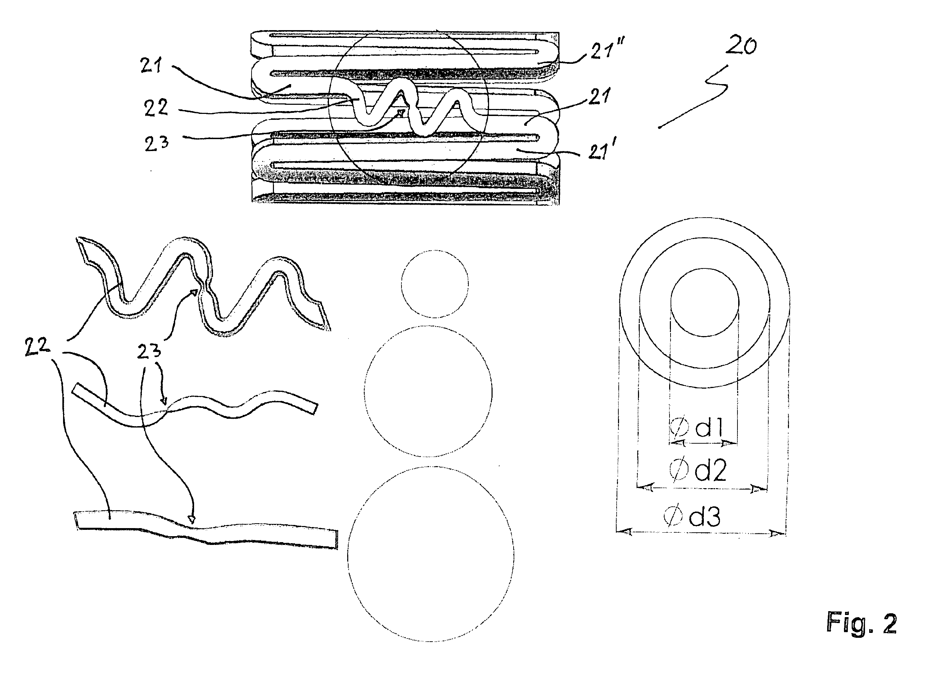

In Fig. 2 sind am Beispiel einer einfachen Ausführungsform mit nur einem einzigen - schlangenlinienförmigen - Dehnelement 22 im Steg 21 des Implantats 20 drei verschiedene Phasen der Aufweitung dargestellt:
Im ursprünglichen, maximal kontrahierten Zustand, in welchem das Implantat 20 in das Gefäß des Patienten eingeführt wird, verlaufen die Schlangenlinien des Dehnelements 22 noch in relativ langen Abschnitten quer zur Längsrichtung der Stege 21, 21' 21". Entsprechend ergibt sich ein minimaler Durchmesser d1 und damit ein minimaler lichter Innenquerschnitt des Implantats 20 an dieser Stelle.

Nach Zuführung von Kraft in radialer Richtung dehnt sich das Dehnelement 22 - seiner Bestimmung gemäß - aus, die Schlangenlinien werden "gestreckt" und verlaufen nunmehr nur noch in kurzen Abschnitten quer zur Längsrichtung. Der Durchmesser d2 und damit der Innenquerschnitt des Implantats 20 werden erheblich vergrößert.

In der dritten Phase schließlich wird das Dehnelement 22 derart gestreckt, dass die Schlangenlinien praktisch zu Geraden werden. Es ergibt sich damit der maximale Durchmesser d3 und damit der größte Innenquerschnitt des Implantats 20. Bei weiterer Krafteinleitung wird dann die als Sollbruchstelle ausgelegte Engstelle 23 definiert brechen.

Das erfindungsgemäße Dehnelement soll aufgrund seiner Materialeigenschaften und seiner Geometrie so ausgebildet sein, dass eine möglichst große Ausdehnung des lichten Querschnitts des röhrenförmigen Implantats in Radialrichtung vom minimal kontrahierten bis zum maximal redilatierten Zustand ermöglicht wird. Realistisch sind Aufweitungen eines ursprünglichen Durchmessers d1 von 1,3mm auf einen Maximaldurchmesser d3 von 18mm oder von d1=2mm auf d3=24mm. In der Praxis wird man sich aber auch schon mit Aufweitungen von d1=3mm auf d3=20mm begnügen können. Auch damit wird schon eine Aufweitbarkeit des Stents auf ein Vielfaches des ursprünglichen Durchmessers erreicht.

Die Herstellung der erfindungsgemäßen Implantate 10; 20, insbesondere der Stege 11,11',11"; 21,21',21" einschließlich der Dehnelemente 12,12'; 22; 32a,32b,32c erfolgt vorzugsweise aus einem rohrförmigen Ausgangsteil durch Laserschneiden.

Vorteilhaft kann es auch sein, wenn das erfindungsgemäße Implantat zumindest abschnittsweise auf seiner Oberfläche eine biologisch aktive Beschichtung, insbesondere eine wachstumshemmende und/oder eine wachstumsfördernde und/oder eine antibakteriell wirkende Beschichtung, erhält.

## Patentansprüche

1. Medizinisches Implantat (10; 20) zur Aufweitung und Stützung eines Körpergefäßes von dessen Innenseite her (="Stent"), das in einem kontrahierten Zustand, vorzugsweise minimal-invasiv, in das Körpergefäß einsetzbar, dort positionierbar und in einen expandierten Zustand überführbar ist, wobei das Implantat (10; 20) röhrenförmig aus länglichen, eine Wand des Implantats (10; 20) bildenden Stegen (11,11',11"; 21,21',21") aufgebaut ist, wobei die Stege (11,11',11"; 21,21',21") zumindest abschnittsweise derart plastisch verformbar sind, dass das Implantat (10; 20) im expandierten Zustand in radialer Richtung quer zur Längsachse der Stege (11,11',11"; 21,21',21") dauerhaft aufgeweitet bleibt, und wobei zumindest einer der Stege (11; 21) ein Dehnelement (12,12'; 22; 32a,32b,32c) aufweist, welches einen Stegabschnitt innerhalb dieses Steges (11; 21) bildet, in dem das Dehnelement (12,12'; 22; 32a,32b,32c) im kontrahierten Zustand teilweise geometrisch quer zur Längsachse des Steges (11; 21) verläuft, wobei der Verlauf des Dehnelements (12,12'; 22; 32a,32b,32c) bezüglich dieser Längsachse mehrere Richtungsänderungen aufweist,
**dadurch gekennzeichet,**
dass Material, Dicke und Stegbreite des Dehnelements (12,12'; 22; 32a,32b,32c) derart gewählt sind, dass durch Krafteinleitung in Radialrichtung des röhrenförmigen Implantats (10; 20) das Dehnelement (12,12'; 22; 32a,32b,32c) durch plastische Streckung in einen dauerhaft expandierten Zustand versetzt werden kann, in welchem der das Dehnelement (12,12'; 22; 32a,32b,32c) bildende Stegabschnitt eine größere geometrische Ausdehnung quer zur Längsachse und eine geringere Ausdehnung in Richtung der Längsachse aufweist als im kontrahierten Zustand,
dass das Dehnelement (12,12'; 22; 32a,32b,32c) im kontrahierten Zustand eine Engstelle (13,13'; 23; 33a,33b, 33c) aufweist, deren Stegbreite geringer ist als die Stegbreiten der anschließenden Stegabschnitte,
dass die Stegbreite der Engstelle (13,13'; 23; 33a,33b,33c) höchstens zwei Drittel so groß ist wie die Stegbreiten der anschließenden Stegabschnitte, und
dass die Engstelle (13,13'; 23; 33a,33b,33c) als Sollbruchstelle für das Dehnelement (12,12'; 22; 32a,32b,32c) ausgelegt ist, die bei zu großer Krafteinleitung in Radialrichtung des röhrenförmigen Implantats (10; 20) bricht, eine weitere Möglichkeit zur Redilatation eröffnet und dadurch eine weitere Expansion des Dehnelements (12,12'; 22; 32a,32b,32c) verhindert.

2. Implantat nach Anspruch 1, **dadurch gekennzeichnet, dass** das Dehnelement (12,12'; 22) aufgrund seiner Materialeigenschaften und seiner Geometrie so ausgebildet ist, dass eine Ausdehnung des Querschnitts des röhrenförmigen Implantats (10; 20) in Radialrichtung vom minimal kontrahierten bis zum maximal redilatierten Zustand von 2mm auf 18mm, vorzugsweise von 4mm auf 16mm, oder von 6mm auf 24mm, vorzugsweise von 6mm auf 20mm, ermöglicht wird.

3. Implantat nach einem der Ansprüche 1 oder 2, **dadurch gekennzeichnet, dass** das Dehnelement (12,12'; 22) im kontrahierten Zustand zumindest abschnittsweise einen zickzackförmigen, schlangenlinienförmigen und/oder mäanderförmigen geometrischen Verlauf aufweist.

4. Implantat nach einem der Ansprüche 1 oder 2, **dadurch gekennzeichnet, dass** das Dehnelement (32a, 32b, 32c) im kontrahierten Zustand zumindest abschnittsweise einen spiralförmigen geometrischen Verlauf aufweist.

5. Implantat nach Anspruch 4, **dadurch gekennzeichnet, dass** das Dehnelement (32a, 32b, 32c) im kontrahierten Zustand zumindest abschnittsweise den geometrischen Verlauf einer Doppelspirale aufweist, die sich ausgehend von einem gemeinsamen Mittelpunkt in zwei entgegengesetzten Drehrichtungen krümmt.

6. Implantat nach einem der Ansprüche 4 oder 5, **dadurch gekennzeichnet, dass** die Spirale eine runde (32b), insbesondere kreisrunde, oder eine dreieckige (32a) oder eine viereckige (32c), insbesondere quadratische Außenkontur aufweist.

7. Implantat nach einem der vorhergehenden Ansprüche, **dadurch gekennzeichnet, dass** die Stegbreite der Engstelle (13,13'; 23; 33a,33b,33c) höchstens halb so groß ist wie die Stegbreiten der anschließenden Stegabschnitte.

8. Implantat nach einem der vorhergehenden Ansprüche, **dadurch gekennzeichnet, dass** zwischen Paaren von in Richtung der Längsachse des Implantats (10) benachbarten Stegen (11', 11") Verbinderelemente (14) angeordnet sind, die bei Krafteinleitung in Längsrichtung dieser Stege (11', 11") eine dauerhafte plastische Vergrößerung des Implantats (10) in axialer Richtung bewirken.

9. Implantat nach einem der vorhergehenden Ansprüche, **dadurch gekennzeichnet, dass** mehrere Dehnelemente (12,12'; 32a,32b, 32c) vorgesehen sind.

10. Implantat nach Anspruch 9, **dadurch gekennzeichnet, dass** die Dehnelemente (12,12') die gleichen mechanischen Eigenschaften aufweisen, insbesondere geometrisch gleich aufgebaut sind.

11. Implantat nach Anspruch 9, **dadurch gekennzeichnet, dass** die Dehnelemente (12,12'; 32a,32b,32c) unterschiedliche mechanischen Eigenschaften aufweisen.

12. Implantat nach Anspruch 11, **dadurch gekennzeichnet, dass** die Dehnelemente (12,12') geometrisch gleich aufgebaut sind, aber unterschiedliche Stegbreiten aufweisen.

13. Implantat nach Anspruch 11, **dadurch gekennzeichnet, dass** die Dehnelemente (32a, 32b, 32c) geometrisch unterschiedlich aufgebaut sind.

14. Implantat nach einem der Ansprüche 9 bis 13, **dadurch gekennzeichnet, dass** mehrere Dehnelemente (12, 12') in Längsrichtung des Implantats (10) hintereinander angeordnet sind.

15. Implantat nach einem der Ansprüche 9 bis 14, **dadurch gekennzeichnet, dass** mehrere Dehnelemente azimutal über den Umfang des Implantats verteilt angeordnet sind.

## Claims

1. Medical implant (10; 20) for widening and supporting a vessel of the body from its inside (="stent"), which can be inserted into the vessel of the body in a contracted state, preferably minimally invasively, can be positioned there and transformed into an expanded state, wherein the implant (10; 20) is designed in the shape of a tube having elongate struts (11, 11', 11"; 21, 21', 21") forming one wall of the (10; 20), wherein the struts (11, 11', 11"; 21, 21', 21") are plastically deformable at least in sections by which the implant implant (10; 20) remains permanently expanded in the radial direction transverse to the longitudinal axis of the struts (11, 11', 11"; 21, 21', 21") and wherein at least one of the struts (11; 21) comprises an expandable element (12, 12'; 22; 32a, 32b, 32c) which forms a strut section within this strut (11; 21) in which the expandable element (12, 12'; 22; 32a, 32b, 32c) in the contracted state runs in part geometrically transverse to the longitudinal axis of the strut (11; 21), wherein the course of the expandable element (12, 12'; 22; 32a, 32b, 32c) has many several changes of direction in relation to this longitudinal axis,
**characterised in that**
the material, thickness and strut width of the expandable element (12, 12'; 22; 32a, 32b, 32c) are selected in such a manner that, by applying force in the radial direction of the tubular implant (10; 20), the expandable element (12, 12'; 22; 32a, 32b, 32c) can be transformed into a permanently expanded condition by plastic elongation, in which the strut section forming the expandable element (12, 12'; 22; 32a, 32b, 32c) has a greater geometrical extension transversely to the longitudinal axis and a smaller extension in the direction of the longitudinal axis than in the contracted state,
the expandable element (12, 12'; 22; 32a, 32b, 32c) has a constriction point (13, 13'; 23; 33a, 33b, 33c) in the contracted state whose strut width is smaller than the strut widths of the adjacent strut sections,
that the strut width of the constriction point (13,13'; 23; 33a, 33b, 33c) is at most two-thirds as large as the strut widths of the adjacent strut sections, and
that the constriction point (13, 13'; 23; 33a, 33b, 33c) is designed as an intended breaking point for the expandable element (12, 12'; 22; 32a, 32b, 32c), which breaks if excessive force is applied in the radial direction of the tubular implant (10; 20), allowing a further opportunity for redilatation and thereby prevents a further expansion of the expandable element (12,12'; 22; 32a, 32b, 32c).

2. Implant in accordance with claim 1, **characterised in that** the expandable element (12,12'; 22), due to its material characteristics and its geometry is so designed that an expansion of the section of the tubular implant (10; 20) in the radial direction from the minimally contracted to the maximally redilated condition from 2mm to 18mm, is enabled and preferably from 4mm to 16mm, or from 6mm to 24mm, preferably from 6mm to 20mm.

3. Implant in accordance with one of claims 1 or 2, **characterised in that** the expandable element (12, 12'; 22) has a zigzag-shaped, sinuously shaped and/or meandering shaped geometric course at least in sections in the contracted condition.

4. Implant in accordance with one of claims 1 or 2, **characterised in that** the expandable element (32a, 32b, 32c) has a spirally shaped geometric course at least in sections in the contracted condition.

5. Implant in accordance with claim 4, **characterised in that** the expandable element (32a, 32b, 32c) has the geometric shape of a double spiral at least in sections in the contracted condition, which curves in two opposing directions of rotation starting from a common midpoint.

6. Implant in accordance with one of claims 4 or 5, **characterised in that** the spiral has an outer contour that is round (32b), in particular circular, or triangular (32a) or quadrangular (32c), in particular square.

7. Implant in accordance with one of the preceding claims, **characterised in that** the strut width of the constriction point (13, 13'; 23; 33a, 33b, 33c) is at most half as large as the strut widths of the adjacent strut sections.

8. Implant in accordance with one of the preceding claims, **characterised in that** connecting elements (14) are arranged between pairs of adjacent struts (11', 11") in the direction of the longitudinal axis of the implant (10), which, when forced is introduced into the longitudinal direction of these struts (11', 11"), induce a permanent plastic expansion of the implant (10) in the axial direction.

9. Implant in accordance with one of the preceding claims, **characterised in that** several expandable elements (12, 12'; 32a, 32b, 32c) are provided.

10. Implant in accordance with claim 9, **characterised in that** the expandable elements (12, 12') have the same mechanical properties and in particular have the same geometrical design.

11. Implant in accordance with claim 9, **characterised in that** the expandable elements (12, 12'; 32a, 32b, 32c) have different mechanical properties.

12. Implant in accordance with claim 11, **characterised in that** the expandable elements (12, 12') have the same geometric design but have different strut widths.

13. Implant in accordance with claim 11, **characterised in that** the expandable elements (32a, 32b, 32c) have different geometric designs.

14. Implant in accordance with one of claims 9 to 13, **characterised in that** several expandable elements (12, 12')are arranged behind one another in the longitudinal direction of the implant (10).

15. Implant in accordance with one of claims 9 to 14, **characterised in that** several expandable elements are distributed azimuthally around the circumference of the implant.

## Revendications

1. Implant médical (10 ; 20) pour l'élargissement et le support d'un vaisseau corporel à partir de son côté intérieur (= « stent ») qui, dans un état contracté, de préférence avec une invasion minimale, peut être introduit dans le vaisseau corporel, y être positionné et être amené dans un état déployé, l'implant (10 ; 20) étant constitué sous une forme tubulaire par des nervures (11, 11', 11" ; 21, 21', 21") oblongues formant une paroi de l'implant (10 ; 20), les nervures (11, 11', 11"; 21, 21', 21") étant, au moins par tronçons, déformables plastiquement de telle sorte que l'implant (10; 20), dans l'état déployé, demeure élargi durablement dans la direction radiale transversalement à l'axe longitudinal des nervures (11, 11', 11"; 21, 21', 21"), et au moins une des nervures (11 ; 21) présentant un élément extenseur (12, 12' ; 22 ; 32a, 32b, 32c) qui forme un tronçon de nervure à l'intérieur de cette nervure (11 ; 21) dans lequel l'élément extenseur (12, 12' ; 22 ; 32a, 32b, 32c), dans l'état contracté, s'étend en partie géométriquement transversalement à l'axe longitudinal de la nervure (11 ; 21), le tracé de l'élément extenseur (12, 12' ; 22 ; 32a, 32b, 32c) présentant plusieurs changements de direction par rapport à cet axe longitudinal,
**caractérisé en ce que**
le matériau, l'épaisseur et la largeur de nervure de l'élément extenseur (12, 12' ; 22 ; 32a, 32b, 32c) sont choisis de telle sorte que, par l'introduction d'une force dans la direction radiale de l'implant de forme tubulaire (10 ; 20), l'élément extenseur (12, 12' ; 22 ; 32a, 32b, 32c) peut être amené par étirement plastique dans un état durablement déployé, dans lequel le tronçon de nervure formant l'élément extenseur (12, 12' ; 22 ; 32a, 32b, 32c) présente une plus grande extension géométrique transversalement à l'axe longitudinal et une plus petite extension dans la direction de l'axe longitudinal que dans l'état contracté,
**en ce que** l'élément extenseur (12, 12' ; 22 ; 32a, 32b, 32c) présente dans l'état contracté un étranglement (13, 13' ; 23 ; 33a, 33b, 33c) dont la largeur de nervure est plus petite que les largeurs de nervure des tronçons de nervure qui suivent,
**en ce que** la largeur de nervure de l'étranglement (13, 13' ; 23 ; 33a, 33b, 33c) est au maximum égale aux deux tiers des largeurs de nervure des tronçons de nervure qui suivent, et
**en ce que** l'étranglement (13, 13'; 23; 33a, 33b, 33c) est conçu en tant qu'emplacement destiné à la rupture pour l'élément extenseur (12, 12' ; 22 ; 32a, 32b, 32c) qui se rompt en cas d'introduction d'une force trop grande dans la direction radiale de l'implant de forme tubulaire (10 ; 20), offre une possibilité supplémentaire de redilatation et empêche de ce fait une expansion supplémentaire de l'élément extenseur (12, 12' ; 22 ; 32a, 32b, 32c).

2. Implant selon la revendication 1, **caractérisé en ce que** l'élément extenseur (12, 12' ; 22), du fait de ses caractéristiques de matériau et de sa géométrie, est constitué de telle sorte qu'une extension de la section transversale de l'implant de forme tubulaire (10 ; 20) dans la direction radiale à partir de l'état contracté au minimum jusqu'à l'état redilaté au maximum est rendue possible de 2 mm à 18 mm, de préférence de 4 mm à 16 mm, ou de 6 mm à 24 mm, de préférence de 6 mm à 20 mm.

3. Implant selon une des revendications 1 ou 2, **caractérisé en ce que** l'élément extenseur (12, 12'; 22) dans l'état contracté présente au moins par tronçons un tracé géométrique en zigzag, sinueux et/ou méandreux.

4. Implant selon une des revendications 1 ou 2, **caractérisé en ce que** l'élément extenseur (32a, 32b, 32c) dans l'état contracté présente au moins par tronçons un tracé géométrique en forme de spirale.

5. Implant selon la revendication 4, **caractérisé en ce que** l'élément extenseur (32a, 32b, 32c) dans l'état contracté présente au moins par tronçons le tracé géométrique d'une double spirale qui, à partir d'un point central commun, se courbe dans deux directions de rotation opposées.

6. Implant selon une des revendications 4 ou 5, **caractérisé en ce que** la spirale présente un contour extérieur rond (32b), en particulier circulaire, ou un contour extérieur triangulaire (32a) ou quadrangulaire (32c), en particulier carré.

7. Implant selon une des revendications précédentes, **caractérisé en ce que** la largeur de nervure de l'étranglement (13, 13' ; 23 ; 33a, 33b, 33c) est au maximum égale à la moitié des largeurs de nervure des tronçons de nervure qui suivent.

8. Implant selon une des revendications précédentes, **caractérisé en ce que**, entre des paires de nervures (11', 11") voisines dans la direction de l'axe longitudinal de l'implant (10), sont disposés des éléments de raccordement (14) qui, lors de l'introduction d'une force dans la direction longitudinale de ces nervures (11', 11 "), provoquent un agrandissement plastique durable de l'implant (10) dans la direction axiale.

9. Implant selon une des revendications précédentes, **caractérisé en ce que** sont prévus plusieurs éléments extenseurs (12, 12' ; 32a, 32b, 32c).

10. Implant selon la revendication 9, **caractérisé en ce que** les éléments extenseurs (12, 12') présentent les mêmes caractéristiques mécaniques, en particulier ils sont de structure géométriquement identique.

11. Implant selon la revendication 9, **caractérisé en ce que** les éléments extenseurs (12, 12' ; 32a, 32b, 32c) présentent des caractéristiques mécaniques différentes.

12. Implant selon la revendication 11, **caractérisé en ce que** les éléments extenseurs (12, 12') sont de structure géométriquement identique mais présentent des largeurs de nervure différentes.

13. Implant selon la revendication 11, **caractérisé en ce que** les éléments extenseurs (32a, 32b, 32c) sont de structure géométriquement différente.

14. Implant selon une des revendications 9 à 13, **caractérisé en ce que** plusieurs éléments extenseurs (12, 12') sont disposés les uns derrière les autres dans la direction longitudinale de l'implant (10).

15. Implant selon une des revendications 9 à 14, **caractérisé en ce que** plusieurs éléments extenseurs sont disposés de façon azimutale en étant répartis sur la circonférence de l'implant.
